# EUROPEAN PATENT APPLICATION

(11) **EP 4 810 087 A1**
(43) Date of publication of application: **23.09.2026**
(21) Application number: 25164926.5
(22) Date of filing: 20.03.2025
(51) Int. Cl.: A61M 15/00, A61M 11/00

(54) **MOUTHPIECE FOR A NEBULIZER, NEBULIZER HAVING THE MOUTHPIECE AND NEBULIZER**

(71) Applicant: PARI Pharma GmbH, 82319 Starnberg (DE)
(72) Inventor: GALLEM, Thomas, 81371 München (DE)
(74) Representative: Hoffmann Eitle

(57) **Abstract**

The present disclosure relates to a mouthpiece (10) for a nebulizer (100), the nebulizer (100) having a boss (126) defining a first aerosol channel (128) and having an outlet (130) and an aerosol generator (114) for ejecting an aerosol into the first aerosol channel (128) towards the outlet (130), the mouthpiece (10) comprising:
a body (12) having a connection portion (16) connectable to the boss (126) of the nebulizer (100) and a delivery opening (22) to be received in the mouth of a user; the body (12) defining a second aerosol channel (14) for transporting the aerosol from the outlet (130) of the boss (126) to the delivery opening (22);
wherein the body (12) defines at least one subchannel (36) separate from the second aerosol channel (14), the at least one subchannel (36) having a first end (38) facing the delivery opening (22) and a second end (40) opposite to the first end (38), wherein a first opening (42) is located at the second end (40) in direct communication with ambience and a second opening (44) is located at the first end (38), wherein the subchannel (36) extends at least in a portion comprising the second opening (44) parallel to second aerosol channel (14).

## Description

### Technical Field

The present disclosure relates to a mouthpiece for a nebulizer, to a nebulizer having the mouthpiece and to a nebulizer.

### Background

Nebulizers for inhalation therapy are known in the art. One type of such a nebulizer is e.g. described in US 2019/0299261 A1 or US 2023/0084119 A1.

The nebulizer comprises a housing, a liquid reservoir defined in the housing, an aerosol generator disposed in the housing for generating an aerosol from a liquid contained in the liquid reservoir, a main channel for receiving and transporting the aerosol from the aerosol generator to a delivery opening and a boss having an outlet and defining a first aerosol channel communicating with the aerosol generator for receiving the aerosol generated by the aerosol generator and transporting the aerosol to the outlet. The nebulizer of US 2019/0299261 A1 further comprises a mouthpiece connected to the boss and including the delivery opening to be received in the mouth of a user. The mouthpiece comprises a body defining a second aerosol channel for transporting the aerosol from the outlet of the boss to the delivery opening. The main channel consists of the first and second aerosol channels.

In US 2023/0084119 A1 opposite side openings are provided in the boss for allowing air (supply air) to enter the first aerosol channel during an inhalation phase and entrain the generated aerosol towards the delivery opening for being inhaled by a user. During exhalation exhaled air (exhaust air) can leave the nebulizer through the side openings so that the delivery opening of the mouthpiece can remain in the mouth of the user throughout the inhalation regimen.

In US 2019/0299261 A1 the mouthpiece body has two top openings for allowing air to enter the second aerosol channel during an inhalation phase and entrain the generated aerosol towards the delivery opening for being inhaled by a user.

The top openings similarly also serve the purpose of allowing air to exit during exhalation with the delivery opening remaining in the mouth of the user.

In either case, the supply air enters the first or second aerosol channel crossing the direction in which the aerosol is ejected from the aerosol generator and, hence, the aerosol flow. Thereby the aerosol flow is biased towards the walls of the first or second aerosol channel. This may lead to increased impaction losses when the aerosol flow impacts on the walls of the first or second aerosol channels. As a result, aerosol performance is decreased.

### Summary

It is one aim of the present disclosure to provide a mouthpiece for a nebulizer, a nebulizer with such a mouthpiece and a nebulizer that enables to minimize impaction losses and to increase aerosol performance.

The basic idea of the present disclosure is to at least additionally feed supply air into the main channel (first or second aerosol channel) or separate from the main channel directly into the mouth of a user so that the central aerosol flow is disturbed as little as possible by cross flows resulting from the supply air. In particular, the supply air is fed into the main channel or separate from the main channel directly into the mouth of a user substantially parallel to the aerosol flow in the main channel.

A mouthpiece according to the present disclosure is defined in claim 1. Further, a nebulizer having such a mouthpiece is defined in claim 6. Furthermore, a nebulizer according to the present disclosure is defined in claim 7. Embodiments of the mouthpiece and the nebulizers are defined in the dependent claims.

According to a first aspect of the present disclosure, a mouthpiece for a nebulizer having a boss defining a first aerosol channel and having an outlet and an aerosol generator for ejecting an aerosol into the first aerosol channel towards the outlet is proposed. Details of the nebulizer, particularly the aerosol generator and the boss may be found further below. Yet, it is to be emphasized already here that the mouthpiece may be used together with any kind of nebulizer including nozzle type nebulizers (also referred to as jet nebulizer), mesh or membrane type nebulizers or ultrasonic type nebulizers.

The mouthpiece of this first aspect comprises a body. The body may be made of one single piece, e.g. in an injection molding process. The body may be made from a polymeric material. According to the first aspect, the body has a connection portion connectable to the boss of the nebulizer.

The connection portion may have a cross-sectional shape corresponding to the cross-sectional shape of the boss. Further, the connection portion may be configured for being friction fitted on or in the boss of the nebulizer. In an example, the connection portion is substantially cylindrical having a circular cross-section. Further, the connection portion may have a connection opening for fitting onto the boss.

The body further has a delivery opening to be received in the mouth of a user. In particular, the body may have a longitudinal extension with the connection portion at one end and the delivery opening at the opposite end. One or more protrusions may, e.g. perpendicularly to the longitudinal extension, protrude from the body to be engaged with the inner side of the teeth of a user so that the mouthpiece can securely be held in the mouth. The protrusions also assist in correctly positioning the mouthpiece in the mouth of a user and may thus, be considered an indicator for the correct position (not to push it too far into the mouth or position it in front of the lips).

Furthermore, the body defines a second aerosol channel for transporting the aerosol from the outlet of the boss to the delivery opening. In a particular embodiment, the second aerosol channel may extend between the connection portion and the delivery opening.

According to the first aspect of the present disclosure, the body defines at least one subchannel separate from the second aerosol channel. The at least one subchannel has a first end facing the delivery opening and a second end opposite to the first end, wherein a first opening is located at the second end in direct communication with ambience and a second opening is located at the first end. "Facing" in this context means that the first end is oriented towards the delivery opening or located on a delivery opening side. It may e.g. be positioned adjacent the delivery opening. To put it differently, the first end is located closer to the delivery opening than the opposite second end. In particular, the subchannel has opposite first and second ends and first and second openings respectively situated at the ends. At least a portion of the subchannel between these two openings is parallel to the second aerosol channel. To put it differently, the (main) flow in the subchannel is at least in a portion of the subchannel parallel to the (main) flow in the second aerosol channel. In particular, the subchannel extends at least in a portion comprising the second opening parallel to second aerosol channel so that ambient air can be supplied to the second aerosol channel parallel to the (main) flow or aerosol flow in the second aerosol channel. A portion of the subchannel comprising the first opening may, however, be angled relative to the second aerosol channel and/or the (main) flow in the second aerosol channel. As a result, ambient air may be introduced into the subchannel in a direction crossing the (main) flow into second aerosol channel. In an alternative, the subchannel may be substantially straight between the first and second openings and extend parallel to the second aerosol channel.

The provision of the at least one subchannel enables that air needed to be introduced into the second aerosol channel in order not to create a negative pressure during the inhalation process is supplied into the second aerosol channel without or at least minimized disturbance of the flow of aerosol ejected from the aerosol generator into the second aerosol channel. In particular, the air may be supplied into the second aerosol channel parallel to the flow of aerosol in the second aerosol channel. Due to these minimized disturbances, impact losses caused by aerosol impacting the inner walls of the body of the mouthpiece and/or the boss can be reduced, and aerosol performance can be improved. During exhalation a certain amount of exhaled air is exhausted via the subchannel/-s. Hence, less air is flowed onto an aerosol generator, such as a membrane, whereby less turbulences are created and less aerosol impacts and, hence, deposits on the inner walls of the mouthpiece.

According to a second aspect of the present disclosure, the second opening opens into the second aerosol channel or is aligned with the delivery opening. In an example, a center axis of the second opening may be parallel to a center axis of the delivery opening. Similar, a center axis of a portion of the subchannel comprising the second opening may have a center axis parallel to the center axis of the delivery opening.

In a further example, the second opening may be arranged so as to be accommodated in the mouth of a user during inhalation and, thus, adjacent or near the delivery opening. In an example comprising the above-mentioned protrusion/-s at the delivery opening to be engaged by teeth and enclosed by the lips of a user, the second opening may be positioned closer to the delivery opening than the protrusion/-s. In the extreme, the second opening is aligned with the delivery opening as mentioned above. In either of these cases, the ambient air is supplied directly into the oral cavity of a user and only mixes with the aerosol exhausted through the delivery opening in the oral cavity. Thus, the oral cavity may also be referred to as a human mixing chamber.

According to a third aspect of the present disclosure, the first opening is distanced from the end of the mouthpiece having the connection portion. Accordingly, the supply air can be introduced into the second aerosol channel close to the delivery opening so that any disturbances of the aerosol flow are limited to a portion close to the delivery opening. This may even further decrease potential losses due to aerosol impacting the inner walls of the body of the mouthpiece.

In an example, the mouthpiece may in its longitudinal direction be separated into at least three sections: a first section comprising the connection portion for being connected to the boss of the nebulizer, a second section only defining a first portion of the second aerosol channel and a third section comprising a second portion of the second aerosol channel as well as the subchannel/-s. In case the second opening of the subchannel is not aligned with the delivery opening, there may even be a fourth section, which may be referred to as a mixing portion into which the second opening and the second aerosol channel opens and in which the aerosol flow and the supply air flow mix.

According to one example, only one subchannel may be provided. In this case, the subchannel may be partially or fully annular and coaxial to the delivery opening.

According to a fourth aspect of the present disclosure, however, at least two of the subchannels are defined in the body. In an embodiment, exactly two such subchannels may be provided.

According to a fifth aspect of the present disclosure, the delivery opening is elliptic. Also, the body of the mouthpiece at its end comprising or defining the delivery opening may be elliptic. In this embodiment, the two subchannels or at least the second openings thereof are arranged at the ends of the major axis of the ellipse in a view perpendicular to the delivery opening. In one example, the subchannels or at least the second openings thereof are arranged in an area of the focal points in a view perpendicular to the delivery opening or along the center axis of the delivery opening. Also, the subchannels may in part follow the outer circumference of the delivery opening and, hence, be partially annular.

As previously mentioned, the present disclosure according to the sixth aspect suggests a nebulizer for delivering an aerosol to a user with the mouthpiece as described above.

The nebulizer of this aspect comprises a housing. Further, the nebulizer comprises a liquid reservoir. The liquid reservoir may be defined in the housing. In this case, the liquid reservoir may comprise a lid and may be filled with liquid upon removing the lid. Alternatively, the liquid reservoir may be receivable by the housing such as an ampoule or container filled with liquid and to be inserted into an optionally opened by the housing.

The nebulizer further comprises an aerosol generator disposed in the housing for generating an aerosol from a liquid contained in the liquid reservoir. The aerosol generator may in general be of any type known in the art, such as membrane or mesh aerosol generator (see below), nozzle type aerosol generator or an ultrasonic type aerosol generator.

Further, the housing may be comprised of several parts including for example a controller portion comprising a control unit of the nebulizer and a nebulizing portion comprising the aerosol generator and/or the liquid reservoir.

The nebulizer further defines a main channel for receiving and transporting the aerosol from the aerosol generator to a/the delivery opening.

The nebulizer further has a boss having an outlet and defining a first aerosol channel communicating with the aerosol generator for receiving the aerosol generated by the aerosol generator and transporting the aerosol to the outlet. The boss may in example be cylindrical in shape and circular in cross-section. The boss may protrude from the housing along its center axis with the outlet facing away from the housing. In addition, a mouthpiece as described above is connected, particularly detachably connected to the boss. In an example, the connection between the mouthpiece and boss is a friction fit. For example a connection portion of the mouthpiece may have an inner diameter (cylindrical inner surface) configured to be fit onto the outer diameter (cylindrical outer surface) of the boss. In the same manner, the connection portion of the mouthpiece may have an outer diameter (cylindrical outer surface) configured to be fit in the inner diameter (cylindrical inner surface) of the boss. In this aspect, the main channel comprises the first and second aerosol channels. In an example, the main channel may even consist of the first and second aerosol channels.

In one example, the boss is not provided with any openings for allowing ambient air to enter the first aerosol channel for the main channel. In this case, supply air can enter the main channel (particularly the second aerosol channel) exclusively via the second opening of the subchannel/-s. Thus, any cross flows relative to the aerosol flow in the main channel can be avoided. Alternatively, it is, however, also possible that the boss is still provided with side openings as in the prior art so that additional supply air is supplied to the main channel, particularly the first aerosol channel via the side openings. Yet, because of the provided subchannel/-s, less air is sucked in via those side openings. Hence, even in this case, the cross flows to the aerosol flow in the main channel, particularly the first aerosol channel, are reduced and the intended effect can to some extent still be achieved.

According to a seventh aspect, the present disclosure also suggests a nebulizer for delivering an aerosol to a user in which the subchannels are either integrated in the boss or the mouthpiece is an integral with the housing of the nebulizer.

Accordingly, the nebulizer of this seventh aspect as well comprises a housing. Further, the nebulizer comprises a liquid reservoir. The liquid reservoir may be defined in the housing. In this case, the liquid reservoir may comprise a lid and be filled with liquid upon removing the lid. Alternatively, the liquid reservoir may be receivable by the housing such as an ampoule or container filled with liquid and to be inserted into an optionally opened by the housing.

The nebulizer further comprises an aerosol generator disposed in the housing for generating an aerosol from a liquid contained in the liquid reservoir. The aerosol generator may in general be of any type known in the art, such as membrane or mesh aerosol generator (see below), nozzle type aerosol generator (jet nebulizer) or an ultrasonic type of aerosol generator.

Further, the housing may be comprised of several parts including for example a controller portion comprising a control unit of the nebulizer and a nebulizing portion comprising the aerosol generator and/or the liquid reservoir.

The nebulizer further defines a main channel for receiving and transporting the aerosol from the aerosol generator to a delivery opening.

The nebulizer further comprises a main channel for receiving and transporting the aerosol from the aerosol generator to a delivery opening.

In addition, at least one subchannel separate from the main channel is provided. The at least one subchannel has a first end facing the delivery opening and a second end opposite to the first end, wherein a first opening is located at the second end in direct communication with ambience and a second opening is located at the first end. "Facing" in this context means that the first end is oriented towards the delivery opening or located on a delivery opening side. It may e.g. be positioned adjacent the delivery opening. To put it differently, the first end is located closer to the delivery opening than the opposite second end. In particular, the subchannel has opposite first and second ends and first and second openings respectively situated at the ends. At least a portion of the subchannel between these two openings is parallel to the main channel. To put it differently, the (main) flow in the subchannel is at least in a portion of the subchannel parallel to the (main) flow in the main channel. In particular, the subchannel extends at least in a portion comprising the second opening parallel to main channel so that ambient air can be supplied to the main channel parallel to the (main) flow or aerosol flow in the main channel. A portion of the subchannel comprising the first opening may, however, be angled relative to the main channel and/or the (main) flow in the main channel. As a result, ambient air may be introduced into the subchannel in a direction crossing the (main) flow into the main channel. In an alternative, the subchannel may be substantially straight between the first and second openings and extend parallel to the main channel.

The provision of the at least one subchannel enables that air, to be introduced into the main channel in order not to create a negative pressure during the inhalation process, is supplied into the main channel without or at least minimized disturbance of the flow of aerosol ejected from the aerosol generator into the main channel. In particular, the air may be supplied into the main channel parallel to the flow of aerosol in the main channel. Due to these minimized disturbances, impact losses caused by aerosol impacting the inner walls of the main channel can be reduced and aerosol performance can be improved.

According to an eighth aspect of the present disclosure, this nebulizer may further comprise a boss having an outlet and defining a first aerosol channel communicating with the aerosol generator for receiving the aerosol generated by the aerosol generator and transporting the aerosol to the outlet, wherein the at least one subchannel is defined by the boss and separate and at least in a portion (please refer to the above definitions) parallel to the first aerosol channel and, hence, the main channel comprising the first aerosol channel. The boss may for example be cylindrical in shape and circular in cross-section. The boss may protrude from the housing along its center axis with the outlet facing away from the housing.

The nebulizer of this aspect further comprises a mouthpiece or face mask connected to, particularly detachably connected to the boss as described with respect to the mouthpiece and boss above. In an example, the connection between the mouthpiece and boss is a friction fit.

The mouthpiece or face mask defines a second aerosol channel and the delivery opening, wherein the main channel comprises or even consists of the first and second aerosol channels.

In one example, the boss is not provided with any openings for allowing ambient air to enter the first aerosol channel for the main channel except for the second opening/s of the subchannel/s. In this case, supply air can enter the main channel (particularly the second aerosol channel) exclusively via the second opening of the subchannel/-s. Thus, any cross flows relative to the aerosol flow in the main channel can be avoided. Alternatively, it is, however, also possible that the boss is still provided with side openings as in the prior art so that additional supply air is supplied to the main channel, particularly the first aerosol channel via the side openings. Yet, because of the provided subchannel/-s, less air is sucked in via those side openings. Hence, even in this case, the cross flows to the aerosol flow in the main channel, particularly the first aerosol channel, are reduced and the intended effect can to some extent still be achieved.

According to this eighth aspect, the subchannel is integrated in the boss rather than in the mouthpiece. Yet, the subchannel can be configured in a similar or even the same way in the boss as in the mouthpiece. In order to avoid repetition, reference is made to the description of the subchannel with respect to the first to fifth aspect of the present disclosure except that subchannel/s is/are arranged in the boss.

A nebulizer of the ninth aspect of the present disclosure further comprises a mouthpiece integrally formed with the housing. In an example, the mouthpiece may be integrally formed with the nebulizing portion of the housing. The term integrally formed in this context is to be understood in that the mouthpiece cannot be removed from the nebulizer without any other essential part of the nebulizer and that the nebulizer is not operable with the combination of the mouthpiece and the other essential part being removed from the nebulizer.

In this embodiment, the mouthpiece has the delivery opening to be received in the mouth of a user and the main channel and the at least one subchannel are defined by the mouthpiece. Hence, in this embodiment, the subchannel is configured in a similar or even the same way as described with respect to the first to fifth aspect of the present disclosure except that the mouthpiece is not detachably connected to the nebulizer. In order to avoid repetition, reference is made to these aspects.

According to a tenth aspect, the second opening opens into the main channel, that is either the first aerosol channel or the second aerosol channel. Alternatively, the second opening may be aligned with the delivery opening.

In an example, a center axis of the second opening may be parallel to a center axis of the delivery opening. Similarly, a center axis of a portion of the subchannel comprising the second opening may have a center axis parallel to the center axis of the delivery opening.

In a further example, the second opening may be arranged so as to be accommodated in the mouth of a user during inhalation and, thus, adjacent or near the delivery opening. In an example comprising the above-mentioned protrusion/-s at the delivery opening to be engaged by teeth of a user, the second opening may be positioned closer to the delivery opening than the protrusion/-s. In the extreme, the second opening is aligned with the delivery opening as mentioned above. In either one of these cases, the ambient air is supplied directly into the oral cavity of a user and only mixes with the aerosol exhausted through the delivery opening in the oral cavity. Thus, the oral cavity may also be referred to as a human mixing chamber.

According to one example, only one subchannel may be provided. In this case, the subchannel may be partially or fully annular and coaxial to the delivery opening.

According to an eleventh aspect of the present disclosure, however, at least two of the subchannels are defined in the body. In an embodiment, exactly two such subchannels may be provided.

According to a twelfth aspect of the present disclosure, the two subchannels or at least the second openings thereof are arranged at the ends of a horizontal axis in a view along a central axis of the main channel. For example, if the main channel is elliptic, the two subchannels or at least the second openings thereof are arranged at the ends of the major axis in a view perpendicular to the delivery opening. In an example, the subchannels or at least the second openings thereof are arranged in an area of the focal points in a view perpendicular to the delivery opening or along the center axis of the delivery opening. Also, the subchannels may in part follow the outer circumference of the delivery opening and, hence, be partially annular.

The aerosol generator of the nebulizer of the thirteenth aspect is a mesh type or membrane type aerosol generator and comprises a vibrateable membrane and a piezoelectric actuator directly acting on the membrane for vibrating the membrane and thereby generating the aerosol.

According to a fourteenth aspect, a distance between the membrane and the second opening is in a range between 30 mm and 70 mm. Thus, a distance between the point where the aerosol is generated and a point where the supply air (ambient air) is supplied to the main channel is sufficiently large in order to minimize the impact losses at the inner walls of the main channel.

According to a fifteenth aspect, no air may pass by the aerosol generator and enter the main channel at its end opposite to the delivery opening. This is to be understood in that no supply air may be introduced into the main channel from behind and by bypassing the membrane. To put it differently, no air can flow through the device past the membrane into the main channel. In an embodiment, the main channel may at its end opposite to the delivery opening be sealingly closed by the aerosol generator such as its membrane.

Finally, we would like to emphasize that even though several features have been described with respect to several aspects, these aspects may also be combined particularly with respect to the more detailed descriptions thereof.

### Brief Description of the Drawings

In the following, the aspects of the present disclosure will be described in more detail with reference to particular embodiments shown in the accompanying drawings.
Fig. 1 shows a mouthpiece according to the present invention in a perspective back view (A), a perspective front view (B) and a longitudinal cross-section (C).
Fig. 2 shows a nebulizer according to the present invention including the mouthpiece of Fig. 1 in a front view (A), a vertical cross-section (B) and a longitudinal cross-section of the mouthpiece (C).
Fig. 3 shows another nebulizer according to the present invention including the mouthpiece of Fig. 1 in a front view (A) and a vertical cross-section (B).
Fig. 4 shows another mouthpiece according to the present invention in a perspective back view (A), a perspective front view (B) and a longitudinal cross-section (C).
Fig. 5 shows another nebulizer according to the present invention including a prior art mouthpiece in a front view (A), a vertical cross-section (B) and a longitudinal cross-section of the mouthpiece (C).

### Detailed Description

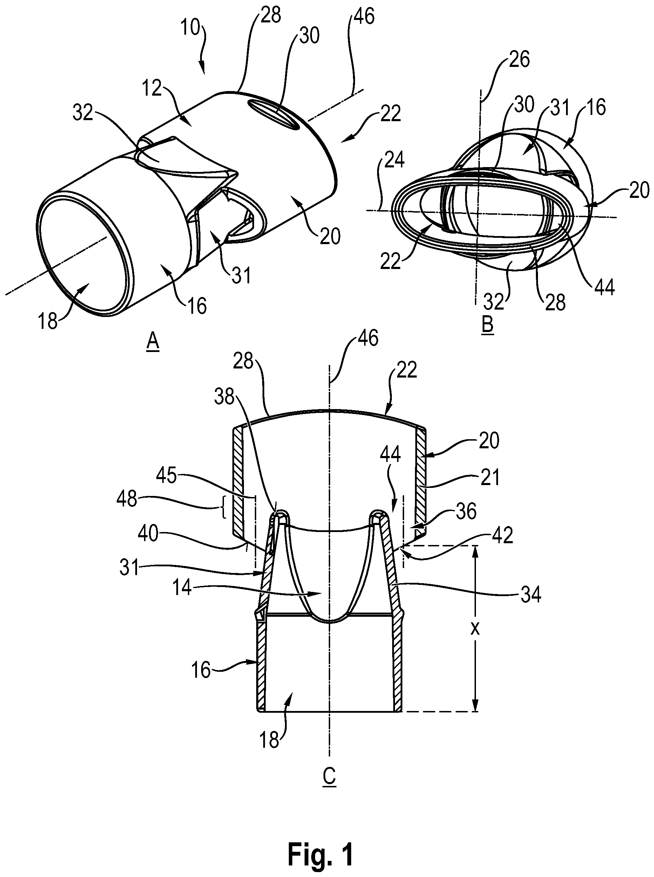

The mouthpiece according to the present disclosure and shown in Fig. 1 comprises a body 12. The body 12 may be formed from a single piece of material. In a particular example, the body 12 may be manufactured in an injection molding process and be made from a polymeric material.

The body 12 defines an aerosol channel (second aerosol channel 14). The second aerosol channel 14 extends in the longitudinal direction of the mouthpiece 10 and the body 12.

The body 12 has opposite ends in its longitudinal direction.

A connection portion 16 is located at one end of the body 12. The connection portion 16 is in the example shown in Fig. 1 cylindrical and has a circular cross-section. A connection opening 18 is located at the longitudinal end of the body 12. As will be explained later, the connection portion 16 with the connection opening 18 allows to detachably mount the mouthpiece to a nebulizer 100.

A delivery portion 20 having a delivery opening 22 is located at the other end of the body 12. In the present example, the delivery opening 22 is elliptic having a major axis 24 and a minor axis 26. In the example, the entire delivery portion 20 is elliptic in cross-section. Further, a center axis of the connection opening 18 and a center axis of the delivery opening 20 are coaxial.

An outer edge 28 defining the delivery opening 22 may be bulged outwardly in top view as best seen in Fig. 1C.

Further, at least one protrusion 30 may be formed adjacent the delivery opening 20 or more particularly the outer edge 28. In the present example, an upper protrusion 30 and a lower protrusion 30 are respectively formed. In the present example, these protrusions 30 are centered on the minor axis 26. The protrusions 30 are to be placed behind the teeth of a user when the mouthpiece 10 or particularly a portion of the delivery portion 20 including the delivery opening 22 are received in the mouth of a user.

A transition portion 31 is formed between the connection portion 16 and the delivery portion 20. The transition portion 31 has upper and lower transition walls 32 for transitioning from the circular cross-section of the connection portion 16 to the elliptic cross-section of the delivery portion 20. In addition, the transition portion 31 has side walls 34 which as best seen in Fig. 1C converge from the connection portion 16 towards the delivery portion 20.

The connection portion 16, the transition portion 31 and the delivery portion 20 together form the second aerosol channel 14.

Further, the body 12 of the mouthpiece 10 defines at least one subchannel 36. In the present example, two such subchannels 36 are provided. In the present example, the subchannels are defined by a portion of the side walls 34 of the transition portion 31 extending into the delivery portion 20 and a portion of the circumferential wall 21 defining the delivery portion 20.

The subchannels 36 have a first end 38 and a second end 40 opposite to the first end 38. A first opening 42, which may also be referred to as inlet opening during inhalation or outlet opening during exhalation, is located at the second end 40. The first opening 42 is in direct communication with ambience. A second opening 44 is located at the first end 38. The second opening 44 may be referred to as supply air opening during inhalation and exhaust air opening during exhalation.

As will be apparent from Fig. 1C, a center axis 45 of the second opening 44 and a center axis 46 of the second aerosol channel 14 are parallel or at least approximately parallel (within ± 15°). Thus, at least the portion 48 of the subchannel comprising the second opening 44 extends parallel to the second aerosol channel 14.

In the embodiment shown in Fig. 1, the second opening 44 opens into the second aerosol channel 14.

Further, the subchannels 36 or at least the second openings 44 are in the example located at the opposite ends of the major axis 24 (at the focal points) of the elliptic delivery opening in a front view on the delivery opening 22, that is a view perpendicular to the delivery opening 22.

The first opening 42 is provided in a distance from the end of the mouthpiece 10 having the connection portion 16. In the particular example, the first opening 42 is arranged in a distance X from the end of the body 10 at which the connection opening 18 is located.

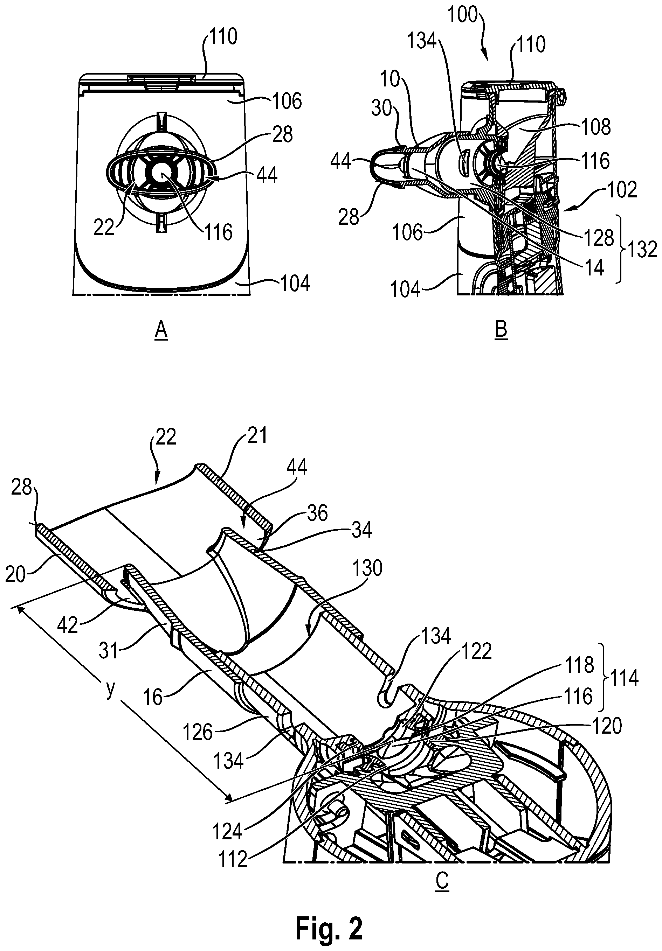

Figure 2 shows the use case of the mouthpiece 10 on a first example of a nebulizer 100.

The nebulizer 100 comprises a housing 102. In the present example, the housing 102 is split into a controller portion 104 and a nebulizing portion 106.

The controller portion 104 may comprise a controller including a printed circuit board and/or a power source, such as a rechargeable battery or battery pack.

The nebulizing portion 106 in the present example comprises a liquid reservoir 108 comprising a lid 110. The lid 110 may be hinged to the remainder of the housing. Upon opening of the lid 110 a liquid may be poured into the liquid reservoir 108. In the present example, the liquid reservoir 108 has an outlet opening 112 at its bottom end.

In other examples, the liquid reservoir 108 may be substituted by an interface for receiving a liquid containing ampoule.

An aerosol generator 114 is also located in the housing 102, particularly, the present example, in the nebulizing portion 106. The aerosol generator 114 has a vibratable membrane 116. A piezoelectric actuator 118 directly acts on the membrane 116 and upon excitation vibrates the membrane 116. In the example, the piezoelectric actuator 118 is directly attached to the vibratable membrane 116.

The vibratable membrane 116 sealingly closes the outlet opening 112 of the liquid reservoir 108. For this purpose, a first sealing lip 120 is interposed between the vibratable membrane 116 and a surface surrounding the outlet opening 112.

The housing 102, particularly the nebulizing portion 106, further defines an ejection opening 122 aligned with an active area of the vibratable membrane 116. A second sealing lip 124 is arranged between a surface surrounding the ejection opening 122 and the vibratable membrane 116 and surrounding the active area of the vibratable membrane 116.

The housing 102 further comprises a boss 126. The ejection opening 122 may be comprised by the boss 126. The boss 126 protrudes from the housing 102. In this context, a center axis of the boss 122 may be slightly inclined upward relative to a horizontal line (when the nebulizer is placed on a horizontal surface).

In the present example, the boss 126 is circular in cross-section and arranged coaxially to the active area of the vibratable membrane 116 and, in the example, the ejection opening 122. The outer diameter of the boss 126 is slightly larger than the inner diameter of the connection portion 16. Accordingly, the mouthpiece 10 may with its connection portion 16 be friction fitted to the outer surface of the boss 126.

The boss 126 has an outlet 130 at its end opposite to its connection to the housing 102 and facing away from the vibratable membrane 116.

The boss 126 defines a first aerosol channel 128 extending from the vibratable membrane 116 and the ejection opening 122 to the outlet 130 of the boss 126.

Thus, with the mouthpiece 10 connected to the boss 126 and, hence, the nebulizer 100, a main channel 132 is defined by the first aerosol channel 128 and the second aerosol channel 14.

Further, as may be seen in Fig. 2C, a distance Y between the vibratable membrane 116 and the second openings 44 is in a range between 30 mm and 70 mm.

Further, the boss 126 has two opposite side openings 134. As will be best visible from Fig. 2C, the side openings 134 are positioned in a location of the boss 126 which is not covered by the connection portion 16 of the mouthpiece 10 when the mouthpiece 10 is connected to the boss 126. The side openings 134 are in direct communication with ambience and open into the first aerosol channel 128. In an embodiment, the cross sectional area of the side openings 134 may in total be equal to the cross sectional area of the first openings 42, such as 56mm². The side openings 134 may be in a range between 30mm² and 110mm². The cross sectional area of the first openings 42 may be in a range between 46mm² and 60mm².

Thus, in use, an aerosol is generated from the liquid in the liquid reservoir 108 by the aerosol generator 114 and ejected into the first aerosol channel 128. In the particular example, the aerosol generator 114 continuously generates an aerosol from the liquid. In other words, the aerosol production is not intermittent and/or triggered for example by the breath of a user.

Upon inhalation, ambient air (supply air) enters the first aerosol channel 128 via the side openings 134 and the second aerosol channel 14 via the subchannels 36. In this regard and due to the parallel orientation of the portion of the subchannels 36, comprising the second opening 44, to the second aerosol channel 14, the supply air is introduced into the second aerosol channel 14 parallel to a main flow in the second aerosol channel 14. Thus, the supply air rather forms an envelope flow around the main flow thus preventing aerosol from impacting the circumferential wall 21 reducing impact losses and, thus, increasing aerosol performance.

In the case the side openings 134 have a larger total cross section of for example 100mm² than the first openings 42 (for example 46 mm²¹, the negative pressure generated by inhalation of a user will be higher in the area of the second openings 44 than the side openings 134. For this reason, the flow rate of supply air entering the main channel is lower at the side openings 134 than at the second openings 44. For this reason, the flow rate of a generated cross flow at the side openings 134 is reduced and impact losses of aerosol impacting the inner surface of the boss 126 can be reduced.

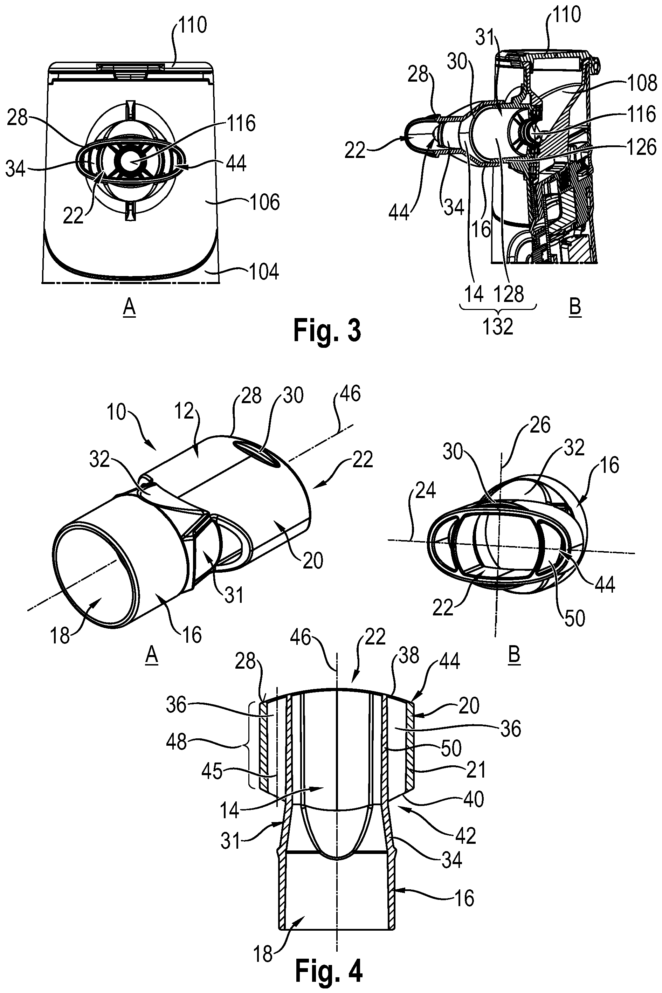

Fig. 3 shows the use case of the mouthpiece 10 on a second example of a nebulizer 100.

The configuration of the nebulizer 100 in Fig. 3 is the same as that shown in Fig. 2 with the difference that the boss 126 has no openings except for the outlet 130. To put it differently, the boss 126 has no side openings 134. Otherwise, the configuration of the nebulizer 100 is the same as that shown in Fig. 3 and a further description is omitted in order to avoid repetition.

Accordingly, during use, supply air is only supplied to the main channel, particularly the second aerosol channel 14 via the subchannels 36 and particularly the second openings 44. Thus, there are even no cross flows relative to the main flow in the main channel, particularly the first aerosol channel and impact losses may even be further reduced.

Fig. 4 shows another embodiment of a mouthpiece 10 in accordance with the present disclosure.

The only difference between the mouthpiece in Fig. 4 and the mouthpiece in Fig. 1 is that the delivery portion 22 has two opposite inner walls 50 respectively defining the two subchannels 36 in combination with the circumferential wall 21. Here, the second openings 44 are aligned with the delivery opening 22. To put it differently, a front edge of the inner walls 50 is flush with the outer edge 28.

Thus, supply air is introduced directly into the mouth of a user rather than into the main channel. Thus, no cross flows at all are generated in the main channel, if the boss 126 has no side openings 134. Thus, impact losses of aerosol impacting the inner walls of the main channel can be reduced. In case the side openings 134 are provided in the boss 126, the same effect as explained above can be achieved with the result of less impact losses.

Otherwise, the mouthpiece 10 in Fig. 4 is the same as that shown in Fig. 1 and further description is omitted in order to avoid repetition.

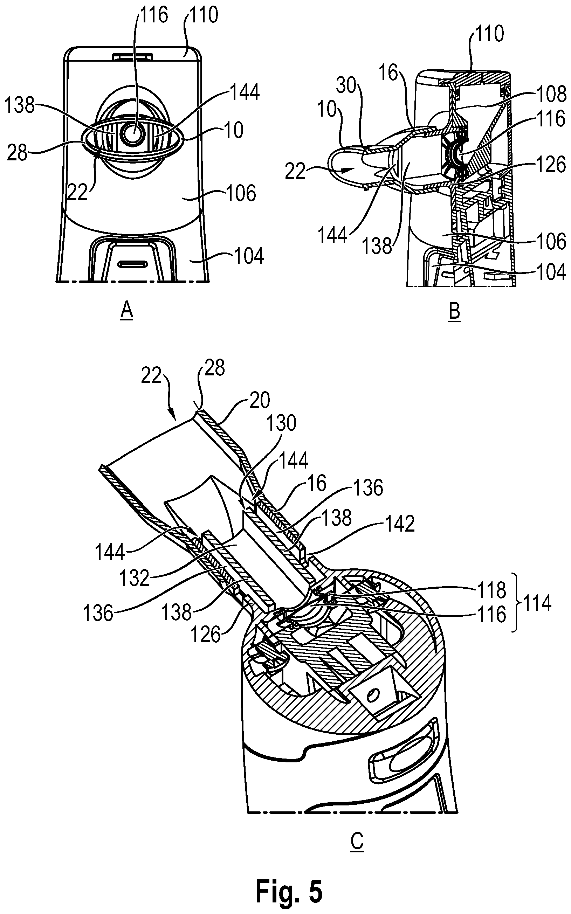

Further, Fig. 5 shows a nebulizer 100 in accordance with the present disclosure and combined with a mouthpiece without subchannels.

The only difference between the nebulizer 100 in Fig. 5 and the nebulizer described above resides in the provision of subchannels 136 in the boss 126.

In particular, the boss 126 still comprises the side openings here representing the first openings 142. However, two inner walls 138 are arranged in the boss 126 and define the subchannels 136. The first openings 142 open into the subchannels 136 at one end (second end) facing the first aerosol channel. The subchannels 136 further have second openings 144 at the opposite end (first end) facing away from the aerosol generator 114. In the present example, the second openings 144 are aligned/flush with the outlet 130. Yet, the inner walls 138 may also end before the edge defining the outlet 130 and, hence, similar to the subchannels 36 in the mouthpiece shown in Fig. 1.

The mouthpiece 10 of this embodiment is a common mouthpiece having all the features of the mouthpiece in Fig. 1 except for the subchannels.

In use of this nebulizer, the same effect may be achieved as with respect to the embodiment shown in Fig. 3. In particular, no cross flows to the main flow are generated but only an envelope flow of supply air entering the main channel via the second openings 144 of the subchannels 136 in the boss 126.

In the above embodiments it has been described that the sub channels are realized by either inner walls 50 of the mouthpiece or inner walls 138 of the boss. Yet, the sub channels may also be formed by a combination thereof, i.e. inner walls of the mouthpiece and inner walls of the boss, which connect to form the sub channel.

### Reference List

- 100: nebulizer
- 102: housing
- 104: controller portion
- 106: nebulizing portion
- 108: liquid reservoir
- 110: lid
- 112: outlet opening
- 114: aerosol generator
- 116: vibratable membrane
- 118: piezoelectric actuator
- 120: first sealing lip
- 122: ejection opening
- 124: second sealing lip
- 126: boss
- 128: first aerosol channel
- 130: outlet
- 132: main channel
- 134: side opening
- 136: subchannel
- 138: inner wall
- 142: first opening
- 144: second opening

- 10: mouthpiece
- 12: body
- 14: second aerosol channel
- 16: connection portion
- 18: connection opening
- 20: delivery portion
- 21: circumferential wall
- 22: delivery opening
- 24: major axis of delivery opening
- 26: minor axis of delivery opening
- 28: outer edge
- 30: protrusion
- 31: transition portion
- 32: transition wall
- 34: side walls
- 36: subchannel
- 38: first end
- 40: second end
- 42: first opening
- 44: second opening
- 45: center axis of the second opening
- 46: center axis of the second aerosol channel
- 48: portion parallel to second aerosol channel
- 50: inner wall

## Claims

1. A mouthpiece (10) for a nebulizer (100), the nebulizer (100) having a boss (126) defining a first aerosol channel (128) and having an outlet (130) and an aerosol generator (114) for ejecting an aerosol into the first aerosol channel (128) towards the outlet (130), the mouthpiece (10) comprising:
a body (12) having a connection portion (16) connectable to the boss (126) of the nebulizer (100) and a delivery opening (22) to be received in the mouth of a user; the body (12) defining a second aerosol channel (14) for transporting the aerosol from the outlet (130) of the boss (126) to the delivery opening (22);
wherein the body (12) defines at least one subchannel (36) separate from the second aerosol channel (14), the at least one subchannel (36) having a first end (38) facing the delivery opening (22) and a second end (40) opposite to the first end (38), wherein a first opening (42) is located at the second end (40) in direct communication with ambience and a second opening (44) is located at the first end (38), wherein the subchannel (36) extends at least in a portion comprising the second opening (44) parallel to second aerosol channel (14).

2. The mouthpiece according to claim 1, wherein the second opening (44) opens into the second aerosol channel (126) or is aligned with the delivery opening (22).

3. The mouthpiece according to claim 1 or 2, wherein the first opening (44) is distanced from the end of the mouthpiece (10) having the connection portion (16).

4. The mouthpiece according to any one of the preceding claims, wherein at least two of the subchannels (36) are defined in the body (12).

5. The mouthpiece according to claim 4, wherein the delivery opening (22) is elliptic and the two subchannels (36) are arranged at the ends of the major axis of the ellipse in a view perpendicular to the delivery opening (22).

6. A nebulizer (100) for delivering an aerosol to a user, the nebulizer comprising:
a housing (102),
a liquid reservoir (108) defined in or receivable by the housing (102),
an aerosol generator (114) disposed in the housing (102) for generating an aerosol from a liquid contained in the liquid reservoir (108),
a main channel (132) for receiving and transporting the aerosol from the aerosol generator (114) to a delivery opening (22);
a boss (126) having an outlet (130) and defining a first aerosol channel (128) communicating with the aerosol generator (114) for receiving the aerosol generated by the aerosol generator (114) and transporting the aerosol to the outlet (130),
a mouthpiece (10) according to any one of the preceding claims connected to the boss (126),
wherein the main channel (132) comprises the first and second aerosol channels (128, 14).

7. A nebulizer (100) for delivering an aerosol to a user, the nebulizer comprising:
a housing (102);
a liquid reservoir (108) defined in or receivable by the housing (102);
an aerosol generator (114) disposed in the housing (102) for generating an aerosol from a liquid contained in the liquid reservoir (108);
a main channel (132) for receiving and transporting the aerosol from the aerosol generator (114) to a delivery opening (22); and
at least one subchannel (136) separate from the main channel (132), the at least one subchannel (136) having a first end (38) facing the delivery opening (22) and a second end (40) opposite to the first end (38), wherein a first opening (142) is located at the second end (40) in direct communication with ambience and a second opening (44) is located at the first end (38), wherein the subchannel (136) extends at least in a portion comprising the second opening (44) parallel to main channel (132).

8. The nebulizer according to claim 7, further comprising
a boss (126) having an outlet (120) and defining a first aerosol channel (128) communicating with the aerosol generator (114) for receiving the aerosol generated by the aerosol generator (114) and transporting the aerosol to the outlet (130), wherein the at least one subchannel (136) is defined by the boss (126) and separate and at least in part parallel to the first aerosol channel (128); and
a mouthpiece or face mask (10) connected to the boss (126), the mouthpiece (10) or face mask defining a second aerosol channel (14) and the delivery opening (22), wherein the main channel (132) comprises the first and second aerosol channels (128, 14).

9. The nebulizer according to claim 7, further comprising
a mouthpiece integrally formed with the housing (102), the mouthpiece having the delivery opening (22) to be received in the mouth of a user, wherein the main channel (132) and the at least one subchannel (136) are defined by the mouthpiece.

10. The nebulizer according to any one of claims 7 to 9, wherein the second opening (44) opens into the main channel (132) or is aligned with the delivery opening (22).

11. The nebulizer according to any one of claims 7 to 10, wherein at least two of the subchannels (136) are defined in the body (102).

12. The mouthpiece according to claim 11, wherein the two subchannels (136) are arranged at the ends of a horizontal axis in a view along a central axis of the main channel (132).

13. The nebulizer according to any one of claims 6 to 12, wherein the aerosol generator (114) comprises a vibratable membrane (116) and a piezoelectric actuator(118) directly acting on the membrane (116) for vibrating the membrane (116) and thereby generating the aerosol.

14. The nebulizer according to claim 13, wherein the distance (Y) between the vibratable membrane (116) and the second opening (44) is in a range between 30 mm and 70 mm.

15. The nebulizer according to any one of claims 6 to 14, wherein no air may pass by the aerosol generator (114) and enter the main channel (132) at its end opposite to the delivery opening.
